# EUROPEAN PATENT SPECIFICATION

(11) **EP 4 561 449 B1**
(45) Date of publication and mention of the grant of the patent: **17.06.2026**
(21) Application number: 23739308.7
(22) Date of filing: 17.07.2023
(51) Int. Cl.: A61B 6/02, G16H 50/30, G16H 30/40, G16H 80/00, G16H 50/00, G16H 30/00, A61B 6/00, A61B 6/42, A61B 6/50

(54) **BLOOD FLOW PARAMETERS**
BLUTFLUSSPARAMETER
PARAMÈTRES DE DÉBIT SANGUIN

(30) Priority: 26.07.2022 EP 22186893
(43) Date of publication of application: 04.06.2025
(73) Proprietor: Koninklijke Philips N.V., 5656 AE Eindhoven (NL)
(72) Inventor: GRASS, Michael, 5656 AG Eindhoven (NL); NICKISCH, Hannes, 5656 AG Eindhoven (NL); HAASE, Christian, 5656 AG Eindhoven (NL); SCHMITT, Holger, 5656 AG Eindhoven (NL); LANGZAM, Eran, 5656AG Eindhoven (NL)
(74) Representative: Philips Intellectual Property & Standards
(86) International application number: PCT/EP2023/069741
(87) International publication number: WO 2024/022855

(56) References cited:
- DE-A1- 102019 218 589
- US-A1- 2018 125 435
- US-A1- 2018 211 386

## Description

### TECHNICAL FIELD

The present disclosure relates to determining a value of a blood flow parameter for one or more vessels. A computer-implemented method, a computer program product, a system, and a processing arrangement, are disclosed.

### BACKGROUND

Various clinical investigations involve an assessment of blood flow in the vasculature. For instance, investigations for coronary artery diseases "CAD" often involve an assessment of blood flow in order to assess the amount of blood supplied to regions of the heart. In this regard, various blood flow parameters have been investigated, including blood flow velocity, blood pressure, the Fractional Flow Reserve "FFR", the instantaneous wave-free ratio "iFR", the Coronary Flow Reserve "CFR", the Thrombolysis in Myocardial Infarction "TIMI" flow grade, the Index of Microvascular Resistance "IMR", the Hyperemic Microvascular Resistance index "HMR". Some blood flow parameters are calculated for individual vessels, whereas others are calculated for a network of vessels.

Historically, values for blood flow parameters such as those mentioned above have been measured using invasive devices such as a pressure-wire. For instance, a value for the Fractional Flow Reserve "FFR" is often determined in order to assess the impact of a stenosis on the delivery of oxygen to the heart muscle in investigations for CAD. The FFR is defined by the ratio *P_{d}*/*Pₐ*, wherein *P_{d}* represents a distal pressure at a distal position with respect to the stenosis, and *Pₐ* represents a proximal pressure with respect to the stenosis. The FFR is typically calculated with values for *P_{d}* and *Pₐ* that are averaged over the cardiac cycle. FFR values above 0.8 are typically regarded as clinically insignificant, and values below 0.8 are typically considered to represent increased clinical significance. Historically, values for these pressures have been determined by positioning an invasive device, such as a pressure wire, at the respective positions in the vasculature.

More recently, angiographic techniques have been developed for determining the values of blood flow parameters, including the FFR. According to fluid flow theory, pressure changes are linked to changes in fluid velocity. In the example of the FFR, angiographic images of an injected contrast agent are analyzed in order to determine the blood flow velocity. The FFR may then be calculated by using a haemodynamic model to estimate pressure values in the vessel from the blood flow velocity.

A known angiographic technique for measuring blood flow velocity is to sample the intensity of an injected contrast agent in computed tomography "CT" angiographic image data over time, and to apply a mathematical model to the sampled data. In this regard, a technique for sampling reconstructed CT images in order to determine blood flow velocity is disclosed in a document by Barfett, J. J., et al., "Intra-vascular blood velocity and volumetric flow rate calculated from dynamic 4D CT angiography using a time of flight technique", Int J Cardiovasc Imaging, 2014, 30:1383-1392. A technique for sampling raw, i.e. "projection" CT data in order to determine blood flow velocity is disclosed in a document by Prevrhal, S. et al., "CT Angiographic Measurement of Vascular Blood Flow Velocity by Using Projection Data", Radiology, Vol. 261: Number 3 - December 2011, pages 923 - 929.

Some angiographic techniques for measuring blood flow parameters involve the use of a computational fluid dynamics "CFD" model. In this regard, a technique for determining an FFR value using a CFD model is disclosed in a document by Taylor. C. A., et al., "Computational Fluid Dynamics Applied to Cardiac Computed Tomography for Noninvasive Quantification of Fractional Flow Reserve: Scientific Basis", JACC, Vol. 61, No. 22, 2013. Another document describing an angiographic technique for measuring blood flow parameters using a computational fluid dynamics "CFD" model is the document by Koo, B. K., et al., "Diagnosis of Ischemia-Causing Coronary Stenoses by Noninvasive Fractional Flow Reserve Computed From Coronary Computed Tomographic Angiograms: Results From the Prospective Multicenter DISCOVER-FLOW (Diagnosis of Ischemia-Causing Stenoses Obtained Via Noninvasive Fractional Flow Reserve) Study", JACC, Vol. 58, No. 19, 2011.

Yet another angiographic technique for measuring blood flow parameters involves the use of a lumped parameter model. A lumped parameter model can be used to represent blood flow in a vascular region as an electrical circuit; a volumetric flow rate of the blood being represented as an electrical current, a pressure of the blood being represented as a voltage, and a blood volume being represented by an electrical charge. In a lumped parameter model, resistance to blood flow is represented by a linear or a non-linear electrical resistor, vessel wall compliance is represented by a capacitor, and blood inertia is represented by an inductor. A machine-learning-based approach for setting the values of linear and non-linear resistors in a static lumped parameter model that is based on angiographically-derived measurements of vessel geometry is disclosed in a document by Nickisch, H., et al., "Learning Patient-Specific Lumped Models for Interactive Coronary Blood Flow Simulations", MICCAI 2015, Part II, LNCS 9350, pp. 433 - 441, 2015, and also in the document WO 2016/001017. Values for the blood flow rate and pressure in the model are then solved in order to determine a value for the FFR for a vessel. As compared to computational fluid dynamics "CFD" models, the use of a lumped parameter model to calculate values of blood flow parameters offers the advantages of a reduction in both computational burden, and model complexity.

US 2018/125435 describes an approach for generating CT image data from an examination region of a patient. At least two sets of projection measurement data assigned to different X-ray energy spectra are acquired and at least two different contrast media are present simultaneously during the acquisition. Additionally a method for analyzing morphological and/or functional parameters assigned to different contrast media in an examination region of a patient is described.

Angiographic techniques for determining values of blood flow parameters typically use CT attenuation data because it provides three-dimensional geometric information on vessels in the vasculature. Spectral CT attenuation data has also been used to determine the values of blood flow parameters. Spectral CT attenuation data includes data from multiple different energy intervals, and the processing of such data permits a distinction between materials that have similar X-ray attenuation values when measured within a single energy interval, and which would be indistinguishable in conventional CT attenuation data. This benefit can be exploited to provide more accurate values for blood flow parameters by improving the distinction between attenuation arising from contrast agent material, and attenuation arising from background materials. However, the calculation of values of blood flow parameters from CT attenuation data is a computationally complex task. Moreover, for a given region of interest, spectral CT attenuation datasets can be significantly larger than their conventional CT counterparts. This exacerbates the challenge of determining values for blood flow parameters from spectral CT attenuation data.

Consequently, there remains room to improve the way in which the values of blood flow parameters are determined from spectral CT attenuation data.

### SUMMARY

According to one aspect of the present disclosure, a computer-implemented method of determining a value of a blood flow parameter for one or more vessels, is provided. The method includes:
receiving, by a host processing system, spectral computed tomography, CT, attenuation data representing an injected contrast agent within an anatomical region including the one or more vessels, the spectral CT attenuation data defining X-ray attenuation within the anatomical region in a plurality of different energy intervals;
identifying, by the host processing system, one or more anatomical structures in the spectral CT attenuation data;
determining a subset of the spectral CT attenuation data for use in calculating the value of the blood flow parameter, based on the identified one or more anatomical structures;
transferring the subset of the spectral CT attenuation data from the host processing system over a communication channel to a remote processing system, the remote processing system being configured to calculate the value of the blood flow parameter from the subset of the spectral CT attenuation data; and
receiving, by the host processing system, the value of the blood flow parameter calculated by the remote processing system.

The subset of the spectral CT attenuation data includes only the spectral CT attenuation data corresponding to the identified one or more anatomical structures; or the subset comprises the spectral CT attenuation data corresponding to the identified one or more anatomical structures, and compressed or default values for the spectral CT attenuation data corresponding to one or more regions outside the identified one or more anatomical structures.

As mentioned above, the calculation of values of blood flow parameters from CT attenuation data is a computationally complex task. In the above method, the use of a remote processing system to calculate the value of the blood flow parameter alleviates the computational burden at the host processing system by using the remote processing system to calculate the value of the blood flow parameter. In the above method, the use of spectral attenuation data to calculate the value of the blood flow parameter, rather than conventional CT data, facilitates improved separation between the attenuation arising from the injected contrast agent, and attenuation arising from other media that may be present in the anatomical region. Consequently, the use of spectral attenuation data in the above method facilitates an accurate calculation of the values of blood flow parameter whilst alleviating the computational burden on the host processing system. The inventors have observed, however, that size of spectral CT datasets presents a challenge when transferring spectral CT data to a remote processing system in order to determine the value of a blood flow parameter at the remote processing system. This is because, for a given region of interest, a spectral CT dataset is significantly larger than its conventional CT counterpart. This is a consequence of spectral CT data representing X-ray attenuation at multiple different energy intervals. Since, in the above method, a subset of the spectral CT attenuation data is transferred from the host processing system to the remote processing system over the communication channel, the remote processing system calculates the value of the blood flow parameter with a reduced demand on the communication channel's bandwidth and/or it calculates the value of the blood flow parameter in a reduced amount of time. Thus, the method facilitates the provision of accurate blood flow parameter values with reduced delay.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of determining a value 110 of a blood flow parameter for one or more vessels 120, in accordance with some aspects of the present disclosure.
Fig. 2 is a schematic diagram illustrating an example of a system 300 for determining a value 110 of a blood flow parameter for one or more vessels 120, in accordance with some aspects of the present disclosure.
Fig. 3 is a schematic diagram illustrating an example of a computer-implemented method of determining a value 110 of a blood flow parameter for one or more vessels 120, in accordance with some aspects of the present disclosure.
Fig. 4 is a schematic diagram illustrating a heart, including an example of a vessel 120, in accordance with some aspects of the present disclosure.
Fig. 5 is a schematic diagram illustrating an example of the operations of identifying S120 one or more anatomical structures 150_{1..i} in spectral CT attenuation data 140, and determining S130 a subset 140_{S} of the spectral CT attenuation data 140 for use in calculating the value 110 of the blood flow parameter, in accordance with some aspects of the present disclosure.
Fig. 6 is a graph illustrating the dependence of the mass attenuation coefficient with X-ray energy for two example materials, iodine and water.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, and in a processing arrangement, in a corresponding manner.

In the following description, reference is made to a method of determining a value of a blood flow parameter for a vessel. In some examples, the vessel is a coronary artery that is disposed within a thoracic anatomical region. However, it is to be appreciated that the coronary artery serves only as an example, and that the vessel may in general be any type of vessel, i.e. an artery, or a vein, or a ventricle. It is also to be appreciated that the thoracic anatomical region serves only as an example of an anatomical region in which the vessel may be located, and that the vessel may in general be located in any anatomical region. For instance, the vessel may be located in anatomical regions such as a cephalic region, an abdominal region, an upper limb region, a lower limb region, and so forth.

Reference is also made herein to examples in which the method is used to determine an FFR value for a vessel. However, it is to be appreciated that the FFR serves only as an example of a blood flow parameter, and that the method disclosed herein may alternatively be used to calculate values of other blood flow parameters. For instance, the method may be used to determine a value of blood flow parameters such as, and without limitation, a blood flow velocity, a blood pressure, a blood flow transit time, a volumetric blood flow value, a measure of blood perfusion, an iFR value, a CFR value, a TIMI flow grade, an IMR value, and an HMR value, a hyperemic stenosis resistance "HSR" value, a zero flow pressure "ZFP" value, and an instantaneous hyperemic diastolic velocity-pressure slope "IHDVPS" value.

Moreover, whilst reference is made herein to examples in which a value of a blood flow parameter is determined for a single vessel, the method and system disclosed herein may alternatively be used to calculate values of a blood flow parameter for multiple vessels. In this regard, individual values of a blood flow parameter may be determined for each of multiple vessels, or a single value of a blood flow parameter may be determined for multiple vessels. For instance, the method disclosed herein may be used to calculate a value for a measure of blood perfused by a network of vessels, and in this case, individual values of the blood flow parameter may be calculated for each of multiple vessels in the network, or a single value of the blood flow parameter may be calculated for the network.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "processing system" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, there remains room to improve the way in which the values of blood flow parameters are determined from spectral CT attenuation data.

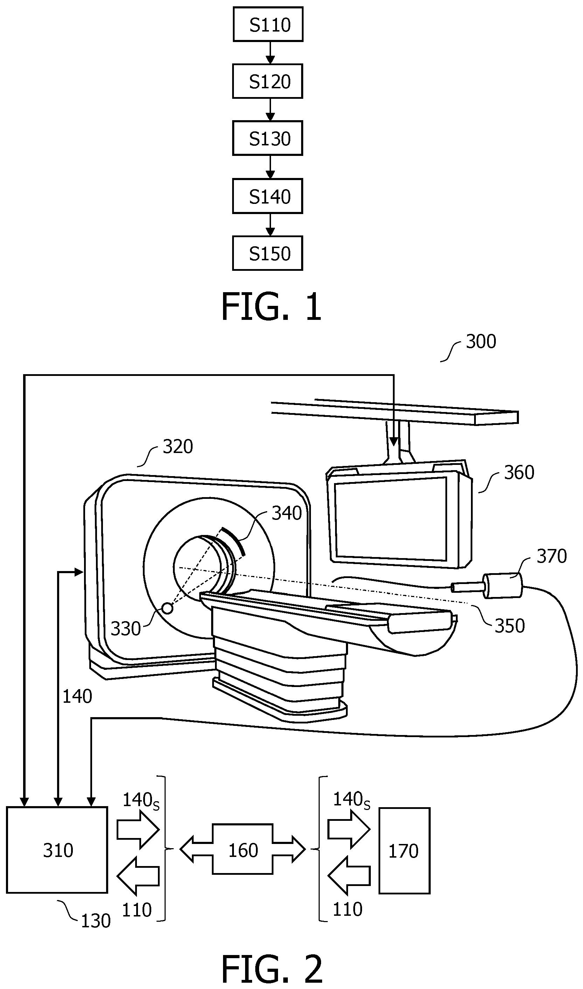

Fig. 1 is a flowchart illustrating an example of a computer-implemented method of determining a value 110 of a blood flow parameter for one or more vessels 120, in accordance with some aspects of the present disclosure. Fig. 2 is a schematic diagram illustrating an example of a system 300 for determining a value 110 of a blood flow parameter for one or more vessels 120, in accordance with some aspects of the present disclosure. Operations described in relation to the method illustrated in Fig. 1, may also be performed by the system 300 illustrated in Fig. 2. Likewise, operations described in relation to the system 300 may also be performed in the method described with reference to Fig. 1. With reference to Fig. 1, the computer-implemented method of determining a value 110 of a blood flow parameter for one or more vessels 120, includes:
receiving S110, by a host processing system 130, spectral computed tomography, CT, attenuation data 140 representing an injected contrast agent within an anatomical region including the one or more vessels 120, the spectral CT attenuation data 140 defining X-ray attenuation within the anatomical region in a plurality of different energy intervals DE_{1..m};
identifying S120, by the host processing system 130, one or more anatomical structures 150_{1..i} in the spectral CT attenuation data 140;
determining S130 a subset 140_{S} of the spectral CT attenuation data 140 for use in calculating the value 110 of the blood flow parameter, based on the identified one or more anatomical structures 150_{1..i};
transferring S140 the subset 140_{S} of the spectral CT attenuation data from the host processing system 130 over a communication channel 160 to a remote processing system 170, the remote processing system being configured to calculate the value 110 of the blood flow parameter from the subset of the spectral CT attenuation data; and
receiving S150, by the host processing system 130, the value 110 of the blood flow parameter calculated by the remote processing system 170.

As mentioned above, the calculation of values of blood flow parameters from CT attenuation data is a computationally complex task. In the above method, the use of a remote processing system to calculate the value of the blood flow parameter alleviates the computational burden at the host processing system by using the remote processing system to calculate the value of the blood flow parameter. In the above method, the use of spectral attenuation data to calculate the value of the blood flow parameter, rather than conventional CT data, facilitates improved separation between the attenuation arising from the injected contrast agent, and attenuation arising from other media that may be present in the anatomical region. Consequently, the use of spectral attenuation data in the above method facilitates an accurate calculation of the values of blood flow parameter whilst alleviating the computational burden on the host processing system. The inventors have observed, however, that size of spectral CT datasets presents a challenge when transferring spectral CT data to a remote processing system in order to determine the value of a blood flow parameter at the remote processing system. This is because, for a given region of interest, a spectral CT dataset is significantly larger than its CT counterpart. This is a consequence of spectral CT data representing X-ray attenuation at multiple different energy intervals. Since, in the above method, a subset of the spectral CT attenuation data is transferred from the host processing system to the remote processing system over the communication channel, the remote processing system calculates the value of the blood flow parameter with a reduced demand on the communication channel's bandwidth and/or it calculates the value of the blood flow parameter in a reduced amount of time. Thus, the method facilitates the provision of accurate blood flow parameter values with reduced delay.

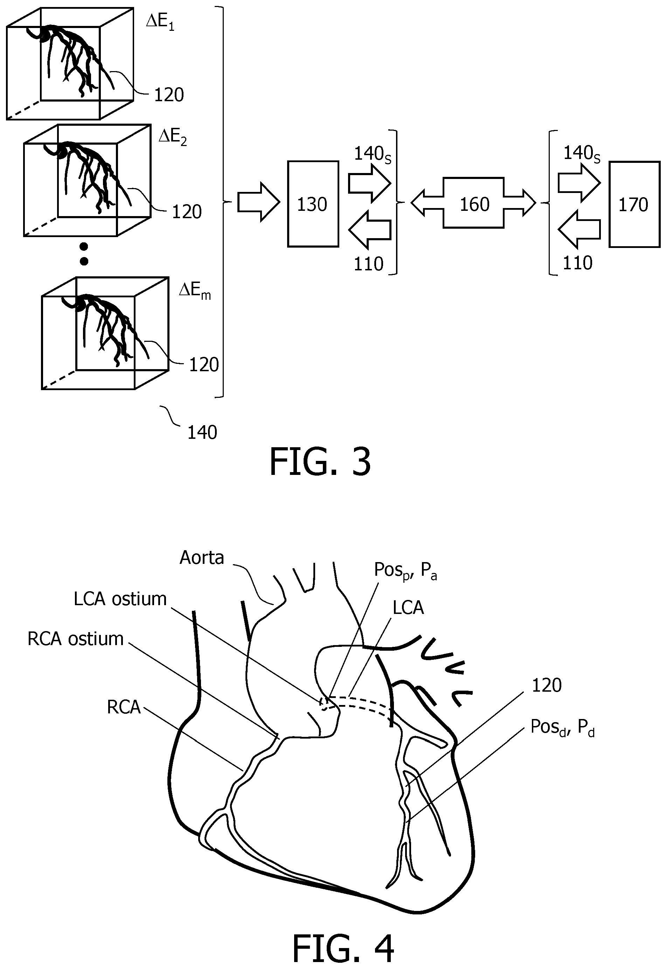

The method illustrated in Fig. 1 is also described with reference to Fig. 3, which is a schematic diagram illustrating an example of a computer-implemented method of determining a value 110 of a blood flow parameter for one or more vessels 120, in accordance with some aspects of the present disclosure.

With reference to Fig. 1 - Fig. 3, in the operation S110, spectral CT attenuation data 140 is received by a host processing system 130. The spectral CT attenuation data represents an injected contrast agent within an anatomical region including one or more vessels 120. The contrast agent may include a substance such as iodine, or a Lanthanide such as gadolinium, or another substance that provides visibility of a blood flow into which the contrast agent is injected. The contrast agent may be injected manually, or by means of an injector such as the injector 370 illustrated in Fig. 2, for example. The anatomical region that is represented by the spectral CT attenuation data 140 may in general be any part of the anatomy that includes one or more vessels. In some examples, the spectral CT attenuation data 110 represents a thoracic anatomical region. The thoracic anatomical region includes anatomical structures such as the heart, the lungs, and so forth, and portions of such anatomical structures may be represented in the spectral CT data 140. Fig. 4 is a schematic diagram illustrating a heart, including an example of a vessel 120, in accordance with some aspects of the present disclosure. The heart illustrated in Fig. 4 is labelled with the left coronary artery, LCA, the right coronary artery, RCA, and the LCA ostium and RCA ostium that respectively define the openings of these arteries in the aorta. The spectral CT attenuation data 140 that is received in the operation S 110 may, inter alia, represent an injected contrast agent in the vessel 120 in the heart illustrated in Fig. 4, i.e. the left coronary artery. As described in more detail below, in some examples of the method illustrated in Fig. 1, the value 110 of a blood flow parameter is calculated for one or more vessels from the spectral CT attenuation data 140. The value of the blood flow parameter may be calculated for the vessel 120 illustrated in Fig. 4, for example.

The spectral CT attenuation data 140 that is received in the operation S 110 may in general be raw data, i.e. data that has not been reconstructed into volumetric image, or it may be image data, i.e. data that represents a reconstructed volumetric image. The spectral CT attenuation data 140 is received by a host processing system 130. This is illustrated on the left-hand side of Fig. 3, and wherein the spectral CT data, which in this example includes reconstructed image data at each of the multiple energy intervals DE_{1..m}, is received by the host processing system 130. The host processing system 130 includes one or more processors 310, as illustrated in the system 300 illustrated in Fig. 2. In general, the host processing system 130, or more specifically, its one or more processors 310 may receive the spectral CT attenuation data 140 from an imaging system, such as from the spectral CT imaging system 320 illustrated in Fig. 2, or from another source, such as a computer readable storage medium, the Internet, or the Cloud, for example. The spectral CT attenuation data 140 may be received via any form of data communication, including wired, optical, and wireless communication. By way of some examples, when wired or optical communication is used, the communication may take place via signals transmitted on an electrical or optical cable, and when wireless communication is used, the communication may take place via RF or optical signals.

The host processing 130 system may in general be provided by any device that includes a processor. For instance, the host processing 130 system may be provided by a computer such as a tablet, or a laptop, or a desktop computer, or a mainframe computer. The host processing device may alternatively be provided in the form of a mobile communication device, such as a mobile telephone.

The spectral CT attenuation data 140 that is received in the operation S 110 defines X-ray attenuation within the anatomical region in a plurality of different energy intervals DE_{1..m}. In this regard, the spectral CT attenuation data 140 may be generated by a spectral CT imaging system. A spectral CT imaging system generates spectral CT data whilst rotating, or stepping, an X-ray source-detector arrangement around an imaging region. Examples of spectral CT imaging systems include cone beam spectral CT imaging systems, photon counting spectral CT imaging systems, dark-field spectral CT imaging systems, and phase contrast spectral CT imaging systems. By way of an example, the spectral CT attenuation data 140 may be generated by the Spectral CT 7500 that is marketed by Philips Healthcare, Best, The Netherlands.

An example of a spectral CT imaging system 320 that may be used to generate the spectral CT attenuation data 140 that is received in the operation S110, is illustrated in Fig. 2. The spectral CT imaging system 320 illustrated in Fig. 2 includes an X-ray source 330 and an X-ray detector 340. The X-ray source 330 and the X-ray detector 340 are mechanically coupled to a gantry (not illustrated in Fig. 2). In operation, the X-ray source 330 and the X-ray detector 340 are rotated by means of the gantry around an axis of rotation 350 whilst acquiring spectral CT attenuation data 140 that defines X-ray attenuation in a region of interest disposed in an imaging region of the imaging system 320. The spectral CT attenuation data 140 obtained from multiple rotational angles around the axis of rotation 350 may subsequently be reconstructed into a volumetric image using various image reconstruction techniques.

The spectral CT attenuation data 140 that is received in the operation S 110 defines X-ray attenuation in a plurality of different energy intervals DE_{1..m}. In general there may be two or more energy intervals; i.e. *m* is an integer, and *m* ≥ 2. The ability to generate data that defines X-ray attenuation data in multiple different energy intervals DE_{1..m} distinguishes a spectral CT imaging system from a conventional CT imaging system. By processing the data from the multiple different energy intervals, a distinction can be made between media that have similar X-ray attenuation values when measured within a single energy interval, and which would be indistinguishable from attenuation data generated by a conventional CT imaging system. In this regard, various different configurations of spectral CT imaging systems may be used to generate the spectral CT attenuation data that is received in the operation S110, some of which are described with reference to Fig. 2.

With reference to the example spectral CT imaging system 320 illustrated in Fig. 2, in general, the X-ray source 330 may be provided by multiple monochromatic sources, or by one or more polychromatic sources, and the X-ray detector 330 may include: a common detector for detecting X-ray radiation across multiple different X-ray energy intervals, or multiple detectors wherein each detector detects X-ray radiation within a different X-ray energy interval DE_{1..m}, or a multi-layer detector in which X-ray radiation within each of multiple different X-ray energy intervals is detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and X-ray detectors may be used in a spectral CT imaging system to generate spectral attenuation data defining X-ray attenuation in a plurality of different energy intervals DE_{1..m}. In general, a discrimination between different X-ray energy intervals may be provided at the X-ray source 330 by temporally switching the X-ray anode potential of a single X-ray source 330, i.e. by "rapid kVp switching", or by temporally switching, or filtering, the emission of X-rays from multiple X-ray sources 330. In such configurations, a common X-ray detector 340 may be used to detect X-rays across multiple different energy intervals, attenuation data for each of the energy intervals DE_{1..m} being generated in a time-sequential manner. Alternatively, a discrimination between different X-ray energy intervals DE_{1..m} may be provided at the X-ray detector 340 by using a multi-layer detector, or a photon counting detector. Such detectors can detect X-rays from multiple X-ray energy intervals DE_{1..m} near-simultaneously, and thus there is no need to perform temporal switching at the X-ray source 330. Thus, a multi-layer X-ray detector 340, or a photon counting X-ray detector 340, may be used in combination with a polychromatic X-ray source 330 to generate spectral attenuation data in a plurality of different energy intervals DE_{1..m}.

Other configurations of the aforementioned X-ray sources 330 and X-ray detectors 340 may alternatively be used in a spectral CT imaging system to provide the desired X-ray attenuation data in a plurality of different energy intervals DE_{1..m}. For example, in a yet further configuration, the need to sequentially switch different X-ray sources 330 emitting X-rays in different energy intervals may be obviated by mounting X-ray source-detector pairs to a gantry at rotationally-offset positions around the axis of rotation 350. In this configuration, each source-detector pair operates independently, and a separation between the X-ray attenuation data for the different energy intervals DE_{1..m} is facilitated by virtue of the rotational offsets of the source-detector pairs. Improved separation between the spectral attenuation data for the different energy intervals DE_{1..m} may be achieved with this configuration by applying an energy-selective filter to the X-ray detector(s) 340 in order to reduce the effects of X-ray scatter.

In another configuration, spectral CT attenuation data 140 in a plurality of different energy intervals DE_{1..m} may be provided by intercepting the X-ray beam in a conventional X-ray imaging system with one or more spectral filters. For example, a single filter may be mechanically moved into, and out of, the X-ray beam generated by a polychromatic X-ray source 330 in order to temporally control the spectrum of X-rays detected by an X-ray detector 340. The filter may for instance transmit only a portion of the spectrum of X-rays emitted by the polychromatic X-ray source 330, and thereby provide spectral attenuation data for a first energy interval when the filter intercepts the beam. When the filter is removed from the beam, spectral attenuation data is provided for a second energy interval, e.g. for the complete spectrum emitted by the polychromatic X-ray source. In so doing, X-ray attenuation data for the energy intervals DE_{1..m} is generated in a time-sequential manner. Multiple filters, each with a different X-ray transmission spectrum, may be sequentially switched into, and out of, the X-ray beam in order to increase the number of energy intervals.

Instead of being generated by a spectral CT imaging system, the spectral CT attenuation data 140 that is received in the operation S110 may alternatively be generated by a spectral X-ray projection imaging system. Spectral X-ray projection imaging systems typically include a support arm such as a so-called "C-arm", also known as a gantry, that supports an X-ray source and an X-ray detector. Spectral X-ray projection imaging systems may alternatively include a support arm with a different shape to this example, such as an O-arm, for example. Spectral X-ray projection imaging systems typically generate projection data with the support arm held in a static position with respect to an imaging region during the acquisition of projection data. The projection data may be used to generate a 2D image. However, spectral X-ray projection imaging systems may also generate spectral CT attenuation data, i.e. data that can be reconstructed into a volumetric image. A spectral X-ray projection imaging system may generate spectral CT attenuation data for a region of interest by rotating its support arm around an axis of rotation and acquiring spectral X-ray projection data for the region of interest from multiple rotational angles around the axis of rotation. The set of spectral X-ray projection data from the multiple rotational angles represents spectral CT attenuation data because this may be reconstructed to provide a volumetric image. Various image reconstruction techniques may be used to reconstruct the set of spectral X-ray projection data from the multiple rotational angles into a volumetric image. These techniques process the set of spectral X-ray projection data in the same manner as spectral CT data that is generated by a spectral CT imaging system. Thus, the spectral CT attenuation data 140 that is received in the operation S110 may alternatively be generated by a spectral X-ray projection imaging system.

Returning to Fig. 1, in the operation S120, one or more anatomical structures 150_{1..i} in the spectral CT attenuation data 140 are identified by the host processing system 130.

An anatomical structure may in general refer to an organ, such as a heart, the lung, a brain, and so forth, or it may refer to a sub-element of an organ. For instance, in some examples, the anatomical structure may be a heart, or an atrium of the heart, a myocardium of the heart, a lumen of at least one of the one or more vessels 120, a wall of one or more of the one or more vessels 120, a vessel plaque of one or more of the one or more vessels 120. The operation of identifying S120 one or more anatomical structures 150_{1..i} in the spectral CT attenuation data, may in general be carried out by performing a segmentation operation on the spectral CT attenuation data 140. Various segmentation techniques are known for this purpose, and these include computer vision methods such as feature clustering, model-based segmentation, the use of feature detectors, as well as trained neural networks.

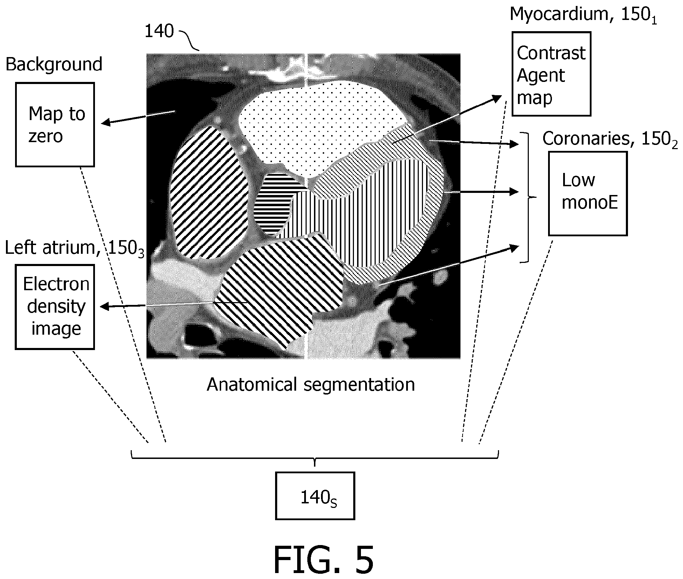

Fig. 5 is a schematic diagram illustrating an example of the operations of identifying S120 one or more anatomical structures 150_{1..i} in spectral CT attenuation data 140, and determining S130 a subset 140_{S} of the spectral CT attenuation data 140 for use in calculating the value 110 of the blood flow parameter, in accordance with some aspects of the present disclosure. The spectral CT attenuation data 140 illustrated in Fig. 5 represents the thoracic region and includes the heart as an example of an anatomical structure. In this example, the sub-elements of the heart: myocardium 150₁, coronary arteries 150₂, and the left atrium 150₃, have been identified using a model-based segmentation of the spectral CT attenuation data 140.

Returning to the method illustrated in Fig. 1, after having identified the one or more anatomical structures 150_{1..i} in the spectral CT attenuation data 140, in the operation S130, a subset 140_{S} of the spectral CT attenuation data 140 is determined for use in calculating the value 110 of the blood flow parameter, based on the identified one or more anatomical structures 150_{1..i}. In general, the operation S130 may be performed by the host processing system 130, or by the remote processing system 170. The subset 140_{S} may be determined based on the identified one or more anatomical structures 150_{1..i} using a look-up table, or a neural network, for example. In such cases, the lookup table, or the neural network, identifies for the one or more anatomical structures 150_{1..i}, a corresponding subset 140s of the spectral CT attenuation data that is required for calculating the value of a blood flow parameter. For instance, with reference to Fig. 5, if the desired blood flow parameter is the FFR, and the anatomical structure is the heart, the lookup table may identify that the subset 140_{S} includes: a contrast agent map for the myocardium 150₁, data from a relatively lower energy interval, "Low monoE", i.e. DE₁ of a dual energy spectral CT imaging system that includes data from two energy intervals DE₁, DE₂, for the coronary arteries, and an electron density image for the left atrium 150₃. The lookup table may similarly identify a subset 140_{S} of the spectral CT attenuation data 140 that is required in order to calculate the value of other blood flow parameters, such as for example a value for the iFR, the CFR, a TIMI flow grade, an IMR value, and an HMR value, a hyperemic stenosis resistance "HSR" value, a zero flow pressure "ZFP" value, and an instantaneous hyperemic diastolic velocity-pressure slope "IHDVPS" value. Similarly, the lookup table may identify a subset 140_{S} of the spectral CT attenuation data 140 that is required for other anatomical regions.

Rather than using a lookup table to identify the subset 140_{S} of the spectral CT attenuation data 140 that is required in order to calculate the value of a desired blood flow parameter, a neural network may be trained to identify the subset 140_{S}. In this case, the neural network may be inputted with an image of the anatomical region, together with input indicative of the desired blood flow parameter, and the neural network may be trained to output the corresponding subset 140_{S} that is required to calculate the value of the blood flow parameter. The training data for this neural network may include multiple images of anatomical regions and a corresponding list of the data this is required to calculate the value of the blood flow parameter. The subset 140_{S} may be determined based on the data that is required by the technique that is used to calculate the value of the blood flow parameter.

Returning to the method illustrated in Fig. 1, in the operation S140, the subset 140_{S} of the spectral CT attenuation data is then transferred from the host processing system 130 over a communication channel 160 to a remote processing system 170. This operation is illustrated in Fig. 2 and Fig. 3. The communication channel 160 may in general be provided by any data communication channel, including a wired communication channel, an optical communication channel, and a wireless communication channel. In other words, when a wired communication channel or an optical communication channel is used, the communication may take place via signals transmitted on an electrical or optical cable, and when a wireless communication channel is used, the communication may take place via RF or optical signals.

In one example, data within the subset 140_{S} of the spectral CT attenuation data is transmitted according to a priority. In this example, the subset 140_{S} of the spectral CT attenuation data comprises a plurality of data elements, the data elements corresponding to different anatomical structures 150_{1..i} and/or different materials in the anatomical region. In this example, the method described with reference to Fig. 1 includes:
assigning, by the host processing system 130, or by the remote processing system 170, a transmission priority to the plurality of data elements in the subset; and
wherein the transferring the subset 140_{S} of the spectral CT attenuation data comprises transferring the data elements in the order of the assigned transmission priority.

The transmission priority may be defined based on factors such as the type of blood flow parameter that is to be calculated, the anatomical region that is represented in the spectral CT data 140, the anatomical structures 150_{1..i} that are identified in the operation S120, and the materials that are comprised in the anatomical structures 150_{1..i}. For instance, if an FFR value is calculated by the remote processing system 170, the host processing system 130 may transfer the subset 140_{S} of the spectral CT attenuation data by prioritizing the transfer of data representing the coronary arteries 150₂ over data representing the myocardium 150₁. This facilitates a more time-efficient transfer of the subset 140_{S} because the remote processing system 170 can begin by processing the data from the most relevant regions, such as the coronary arteries 150₂, without waiting for the transfer of data for other anatomical structures such as the myocardium 150₁. By prioritizing the transfer of the subset 140_{S} in this manner, the time required to compute the value of the blood flow parameter may be reduced.

The transmission priority may be assigned by the host processing system 130, or by the remote processing system 170. In the latter case, the remote processing system 170 may operate in a "pull" / "on-demand" mode wherein the remote processing system 170 uses the result of processing a current data element, e.g. the result of processing data for the coronary arteries 150₂, to identify a subsequent data element that is required by the remote processing system 170 to calculate the value 110 of the blood flow parameter.

The remote processing system then calculates the value 110 of the blood flow parameter from the subset 140_{S} of the spectral CT attenuation data. In this regard, the various techniques cited above may be used to calculate the value 110 of the blood flow parameter. For instance, the technique for sampling reconstructed CT images in order to determine blood flow velocity disclosed in the document cited above by Barfett, J. J., et al., may be used, or the technique for sampling raw, i.e. "projection" CT data in order to determine blood flow velocity disclosed in the cited document by Prevrhal, S. et al., may be used. Other techniques such as the technique for determining an FFR value using a CFD model is disclosed in the cited document by Taylor. C. A., et al., or the angiographic technique for measuring blood flow parameters using a computational fluid dynamics "CFD" model in the cited document by Koo, B. K., et al., or the lumped parameter model in the cited document by Nickisch, H., et al., and in WO 2016/001017, may be used to determine an FFR value for a vessel. Such techniques can be computationally complex, and so by using the remote processing system to calculate the value 110 of the blood flow parameter rather than the host processing system 130, the processing burden on the host processing system 130 is alleviated. Moreover, since a subset 140_{S} of the spectral CT attenuation data 140 is transferred to the remote processing system 170 instead of the complete set of spectral CT attenuation data 140, the remote processing system 170 calculates the value of the blood flow parameter with a reduced demand on the communication channel's bandwidth and/or it calculates the value of the blood flow parameter in a reduced amount of time. Thus, the method facilitates the provision of blood flow parameter values with reduced delay.

The value 110 of the blood flow parameter that is calculated by the remote processing system 170 is then transferred over the communication channel 160 to the host processing system 130, and in the operation S150, the value 110 of the blood flow parameter that was calculated by the remote processing system 170 is received by the host processing system 130.

The value 110 of the blood flow parameter may then be outputted by the host processing system 130. The value 110 may be outputted in various ways. For example, the value of the blood flow parameter may be outputted to a display device such as the monitor 370 of the system 300 illustrated in Fig. 2. Alternatively, the value may be outputted to a computer readable storage medium, or to a printer device, for example. In some examples, the value 110 of the blood flow parameter is outputted as a numerical value, and in other examples, the value 110 of the blood flow parameter may be outputted graphically. For instance, the value of the blood flow parameter may be outputted as a color-coded icon. The value of the blood flow parameter may also be calculated at multiple positions along a length of the vessel 120 and outputted graphically. For example, an image illustrating the vessel 120 may be outputted with the values of the blood flow parameter displayed at corresponding positions along the length of the vessel as a color-coded overlay on the image. A physician may then use the provided value 110 of the blood flow parameter to perform a clinical diagnosis on a subject.

As described above, in the operation S130, a subset 140_{S} of the spectral CT attenuation data (140) is determined based on the identified anatomical structure(s) 150_{1..i}. In this regard, the identified anatomical structure(s) 150_{1..i} may be used in various ways to determine the subset 140_{S}. In one example, the subset 140s of the spectral CT attenuation data includes only the spectral CT attenuation data corresponding to the identified one or more anatomical structures 150_{1..i}. With reference to Fig. 5, in this example, the subset 140_{S} of the spectral CT attenuation data may for example only include the spectral CT attenuation data corresponding to the heart, or to the myocardium 150₁, or to the coronary arteries 150₂, or to the left atrium 150₃, or to a combination of these anatomical structures 150_{1..3}. By omitting the spectral CT attenuation data 140 for other anatomical structures, the amount of data that is transferred in the operation S140 is reduced.

In another example, the subset 140_{S} of the spectral CT attenuation comprises the spectral CT attenuation data corresponding to the identified one or more anatomical structures 150_{1..i} that were identified in the operation S120, and compressed or default values for the spectral CT attenuation data corresponding to one or more regions outside the identified one or more anatomical structures 150_{1..i}. In this example, data for a background region that is outside the identified one or more anatomical structures 150_{1..i} may be converted to default values, such as zero intensity values, as illustrated in the upper left portion of Fig. 5. Alternatively, compressed values may be provided for regions such as this background region. The compressed values may represent the spectral CT attenuation data corresponding to the one or more regions outside the identified one or more anatomical structures 150_{1..i} with a relatively lower spatial resolution than the spectral CT attenuation data corresponding to the identified one or more anatomical structures 150_{1..i}. This may be achieved by mapping the image intensity values of groups of image pixels in the background region to an average value for the group, for example. The compressed values may alternatively represent the spectral CT attenuation data corresponding to the one or more regions outside the identified one or more anatomical structures 150_{1..i} with a relatively lower bit depth than the spectral CT attenuation data corresponding to the identified one or more anatomical structures 150_{1..i}. This may be achieved by quantizing the image intensity values of pixels in the background region using fewer threshold levels, for example. These examples therefore reduce the amount of data for the background region that is transferred over the communication channel 160 in the operation S140, and this reduces the demand on its bandwidth.

The identified anatomical structure(s) 150_{1..i} may be used in other ways to determine the subset 140_{S} of the spectral CT attenuation data. In another example, the subset 140_{S} of the spectral CT attenuation data corresponds to the one or more anatomical structures 150_{1..i}, and the subset 140_{S} of the spectral CT attenuation data represents at least one of the one or more anatomical structures 150_{1..i} with a subset of the plurality of energy intervals DE_{1..m}. For instance, with reference to Fig. 5, in this example, the subset 140_{S} of the spectral CT attenuation data may correspond to the coronary arteries 150₂, and in this case, the subset 140_{S} may represents the coronary arteries 150₂ with a subset of the energy intervals DE_{1..m}. Since, in this example, the data for the coronary arteries does not include data for all of the energy intervals DE_{1..m}, a reduced amount of data for the coronary arteries is transferred over the communication channel 160 in the operation S140, and this reduces the demand on its bandwidth.

In another example, the subset 140_{S} of the spectral CT attenuation data corresponds to the one or more anatomical structures 150_{1..i} that are identified in the operation S120, and the spectral CT attenuation data represents a plurality of different materials in the anatomical region. The subset 140_{S} of the spectral CT attenuation data represents at least one of the one or more anatomical structures 150_{1..i} with a subset of the materials represented by the spectral CT attenuation data. In this example, the spectral CT attenuation data may for instance represent iodine contrast agent, bone, soft tissue, and water, in the anatomical region. The anatomical structures 150_{1..i} that are identified may include the myocardium 150₁, the coronary arteries 150₂, and the left atrium 150₃. Rather than transferring data for all of the materials represented in each of these anatomical structures, in this example, at least one of the anatomical structures, e.g. the coronary arteries, may be represented using only the data for a contrast agent material, iodine. Since, in this example, the data for other materials, such as soft tissue, is omitted for the coronary arteries, the amount of data that is transferred in the operation S 140 is reduced.

In the above examples, the materials may be identified by applying a material decomposition algorithm to the spectral CT attenuation data 140.

One example of a material decomposition technique that may be used to identify the materials is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another suitable material decomposition technique is disclosed in a document by Roessl, E. and Proksa, R., "K-edge imaging in x-ray computed tomography using multi-bin photon counting detectors", Phys Med Biol. 2007 Aug 7, 52(15):4679-96. Another suitable material decomposition technique is disclosed in the published PCT patent application WO/2007/034359 A2. Another suitable material decomposition technique is disclosed in a document by Alvarez, et al., "Energy-selective reconstructions in X-ray computerized tomography", Physics in medicine and biology, Vol. 21, No. 5, page 733 - 744.

In general, the X-ray attenuation spectrum of a material includes a contribution from Compton Scatter and a contribution from the Photo-electric effect. The attenuation due to Compton scatter is relatively similar for different materials, whereas the attenuation from the Photo-electric effect is strongly material-dependent. Both Compton Scatter and the Photo-electric effect exhibit an energy dependence, and it is this effect that is exploited by material decomposition techniques in order to distinguish between different materials.

In general, material decomposition algorithms operate by decomposing the attenuation spectrum of an absorbing medium into contributions from an assumed set of "basis" materials. The energy-dependent X-ray attenuation of the assumed basis materials is typically modelled as a combination of absorption from Compton Scatter, and the Photo-electric effect. Some materials also have a k-absorption edge "k-edge" energy that is within the energy range used by diagnostic X-ray imaging systems, and this effect may also be exploited in order to distinguish between different materials. The spectral decomposition algorithm then seeks to estimate an amount of each of the basis materials that is required to produce the measured amount of X-ray attenuation at the two or more energy intervals. Water and iodine are examples of basis materials that are often separated in clinical practice using a so-called two-material decomposition algorithm. Non-fat soft tissue, fat, and iodine, are examples of basis materials that are often separated in clinical practice using a three-material decomposition algorithm.

As mentioned above, if the k-absorption edge "k-edge" energy of any of the basis materials is within the energy range used by diagnostic X-ray imaging systems, i.e., approximately 30 - 120 keV, this can be exploited in order to help identifying the contribution of a basis material to X-ray attenuation. The k-edge energy for a material is defined as the minimum energy required for the Photo-electric event to occur with a k-shell electron. The k-edge occurs at a characteristic energy for each material. The k-edge energy for a material is marked by a sharp increase in its X-ray attenuation spectrum at X-ray energies corresponding to the k-edge energy value. The k-edge energies of many materials that are present in the human body are too low to be detected in a diagnostic X-ray imaging system. For example, the k-edge energies of hydrogen, carbon, oxygen, and nitrogen are at energies that are less than 1 keV. However, materials such as iodine (k-edge = 33.2 keV), gadolinium (50.2 keV), gold (80.7 keV), platinum (78.4 keV), tantalum (67.4 keV), holmium (55.6 keV), and molybdenum (k-edge = 20.0 keV) have k-edge energy values that permit their distinction in the spectral CT attenuation data acquired from diagnostic X-ray imaging systems.

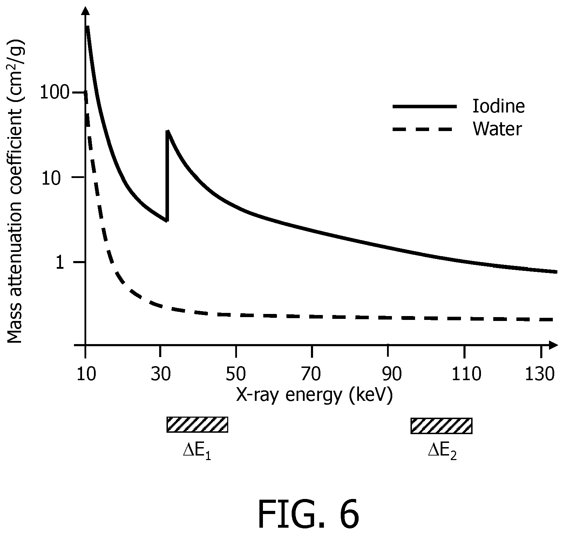

By way of an example, Fig. 6 is a graph illustrating the dependence of the mass attenuation coefficient with X-ray energy for two example materials, iodine and water. The mass attenuation coefficient represented in Fig. 5 represents the X-ray attenuation. The sharp increase in X-ray attenuation for iodine at 33.2 keV facilitates a separation between the contributions of each of these materials in a combined attenuation spectrum that includes attenuation arising from both of these materials. For instance, the X-ray attenuation arising from iodine and water may be separated by using one energy interval DE₁ that is close to the k-edge energy of 33.2 keV, and another energy interval DE₂ that is significantly above the k-edge energy.

One or more additional operations may also be performed in the method described above with reference to Fig. 1. For instance, in one example, the remote processing system 170 is further configured to:
predict a confidence value representing an expected accuracy of the calculated value 110 of the blood flow parameter; and
transfer the confidence value to the host processing system 130 over the communication channel 160.

The confidence value may be calculated based on e.g. an amount of noise in the subset 140_{S}. The confidence value may be outputted by the host processing system 130. The confidence value may be outputted as error bars on the calculated value 110 of the blood flow parameter, for example. A physician may use to confidence value of determine how much reliance to place on the value of the blood flow parameter.

In another example, the remote processing system 170 is configured to:
identify a type of the data in the subset 140_{S} of the spectral CT attenuation data transferred to the remote processing system; and
select an algorithm for use by the remote processing system 170 to calculate the value 110 of the blood flow parameter, based on the identified type of the data; or
translate the subset 140_{S} of the spectral attenuation CT data transferred to the remote processing system 170 into a format for use by the remote processing system to calculate the value 110 of the blood flow parameter, based on the identified type of the data.

In this example, the remote processing system 170 may for example analyze the data in the subset 140_{S} to determine the anatomical structure to which the transferred data pertains, and use the anatomical structure to select an algorithm to use to process the data. For instance, if the result of the analysis indicates that the transferred data relates to the coronary arteries, a first algorithm may be used to calculate the value of the blood flow parameter. By contrast, if the result of the analysis indicates that the transferred data relates to a peripheral artery, a second algorithm may be used to calculate the value of the blood flow parameter. The remote processing system 170 may analyze the data in the subset 140_{S} and determine the anatomical structure to which the transferred data pertains by segmenting the received data, applying a feature detector to the segmented data, for example. Similarly, if the desired blood flow parameter is an FFR value, the result of the analysis may be used to identify the most appropriate algorithm to use to determine its value based on the type of the data that has been transferred. This facilitates the calculation of accurate values for the blood flow parameter.

The remote processing system may alternatively translate the subset 140_{S} based on the identification of the type of data in the subset 140_{S}. In this case, the remote processing system 170 may for example analyze the transferred data to determine a type of the basis function images that have been transferred to the remote processing system 170, and having identified the type of basis function images, translate the subset 140_{S} by e.g. mapping data for a basis function representing soft tissue to a different material, or to different energy intervals that are required by an algorithm to calculate the value of the blood flow parameter. The mapping of this data may be performed based on physical models, or using image to image translation with the help of trained (convolutional) neural network. This also facilitates the calculation of accurate values for the blood flow parameter.

In another example, the method described with reference to Fig. 1 also includes:
receiving, by the host processing system 130, input identifying the one or more vessels 120 in the spectral CT attenuation data 140; and
wherein the value 110 of the blood flow parameter is calculated for the identified one or more vessels 120.

In this example, the operation of identifying the vessel(s) 110 may be performed in an image that is reconstructed from the spectral CT attenuation data 140, or alternatively in an image representing the anatomical region that is reconstructed from conventional CT attenuation data. The input identifying the vessel(s) 180 in the region of interest 120 may be provided by a user, or it may be provided automatically. User input identifying the vessel(s) 180 may be received from a user input device operating in combination with a displayed image of the region of interest. Input identifying the vessel(s) 120 may be provided automatically by using a feature detector, or a trained neural network, to identify the vessel(s) 120 in the spectral CT attenuation data 140. A segmentation operation may also be performed on such images in order to assist in identifying the vessel. Various segmentation algorithms are known for this purpose. A combination of these approaches may also be used in which the feature detector, or the trained neural network, automatically identifies a candidate vessel, and which is then selectable by a user via a user input device. The vessel may be identified based on a detection of contrast agent in such images, or based on the presence of an anomaly, such as a stenosis in the vessel. By way of an example, a feature detector, or a trained neural network may be used to identify the left coronary artery of a cardiac image, and also a stenosis therein, and to identify the vessel as a candidate vessel.

Thus, in this example, the input identifying the one or more vessels 120 may be received from a user input device, or from a processor that executes the operations of the feature detector, or the operations of the trained neural network.

In another example, a computer program product, is provided. The computer program product comprises instructions which when executed by one or more processors 310, cause the one or more processors to carry out a method of determining a value 110 of a blood flow parameter for one or more vessels 120, comprising:
receiving S110, by a host processing system 130, spectral computed tomography, CT, attenuation data 140 representing an injected contrast agent within an anatomical region including the one or more vessels 120, the spectral CT attenuation data 140 defining X-ray attenuation within the anatomical region in a plurality of different energy intervals DE_{1..m};
identifying S120, by the host processing system 130, one or more anatomical structures 150_{1..i} in the spectral CT attenuation data 140;
determining S130 a subset 140_{S} of the spectral CT attenuation data 140 for use in calculating the value 110 of the blood flow parameter, based on the identified one or more anatomical structures 150_{1..i};
transferring S140 the subset 140_{S} of the spectral CT attenuation data from the host processing system 130 over a communication channel 160 to a remote processing system 170, the remote processing system being configured to calculate the value 110 of the blood flow parameter from the subset of the spectral CT attenuation data; and
receiving S150, by the host processing system 130, the value 110 of the blood flow parameter calculated by the remote processing system 170.

In another example, a system 300 for determining a value 110 of a blood flow parameter for one or more vessels 120, is provided. The system comprising one or more processors 310 configured to:
receive S110 spectral computed tomography, CT, attenuation data 140 representing an injected contrast agent within an anatomical region including the one or more vessels 120, the spectral CT attenuation 140 data defining X-ray attenuation within the anatomical region in a plurality of different energy intervals DE_{1..m};
identify S120 one or more anatomical structures 150_{1..i} in the spectral CT attenuation data 140;
determine S130 a subset 140_{S} of the spectral CT attenuation data 140 for use in calculating the value 110 of the blood flow parameter, based on the identified one or more anatomical structures 150_{1..i};
transfer S140 the subset 140_{S} of the spectral CT attenuation data over a communication channel 160 to a remote processing system 170, the remote processing system being configured to calculate the value 110 of the blood flow parameter from the subset of the spectral CT attenuation data; and
receive S150 the value 110 of the blood flow parameter calculated by the remote processing system 170.

An example of the system 300 is illustrated in Fig. 2. It is noted that the system 300 may also include one or more of: a spectral CT imaging system 320 for generating the spectral CT attenuation data 140 that is received in the operation S110; a monitor 360 for displaying the calculated value 110 of the blood flow parameter, and other data described in accordance with the method, an injector 370 for injecting a contrast agent into the vasculature, a patient bed, and a user input device (not illustrated in Fig. 2) that is configured to receive user input for use in conjunction with the method, such as a keyboard, a mouse, a touchscreen, and so forth.

In another example, a processing arrangement for determining a value 110 of a blood flow parameter for one or more vessels 120, is provided. The processing arrangement includes the system 300 described above, and the remote processing system 170.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 300, in a corresponding manner. It is to be understood that a feature described in relation to any one example may be used alone, or in combination with other described features, and may be used in combination with one or more features of another of the examples, or a combination of other examples. Furthermore, modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of determining a value (110) of a blood flow parameter for one or more vessels (120), the method comprising:
receiving (S110), by a host processing system (130), spectral computed tomography, CT, attenuation data (140) representing an injected contrast agent within an anatomical region including the one or more vessels (120), the spectral CT attenuation data (140) defining X-ray attenuation within the anatomical region in a plurality of different energy intervals (DE_{1..m});
identifying (S120), by the host processing system (130), one or more anatomical structures (150_{1..i}) in the spectral CT attenuation data (140);
determining (S130) a subset (140_{S}) of the spectral CT attenuation data (140) for use in calculating the value (110) of the blood flow parameter, based on the identified one or more anatomical structures (150_{1..i});
transferring (S140) the subset (140s) of the spectral CT attenuation data from the host processing system (130) over a communication channel (160) to a remote processing system (170), the remote processing system being configured to calculate the value (110) of the blood flow parameter from the subset of the spectral CT attenuation data; and
receiving (S150), by the host processing system (130), the value (110) of the blood flow parameter calculated by the remote processing system (170);
wherein:
the subset (140_{S}) of the spectral CT attenuation data includes only the spectral CT attenuation data corresponding to the identified one or more anatomical structures (150_{1..i}); or
the subset (140_{S}) comprises the spectral CT attenuation data corresponding to the identified one or more anatomical structures (150_{1..i}), and compressed or default values for the spectral CT attenuation data corresponding to one or more regions outside the identified one or more anatomical structures (150_{1..i}).

2. The computer-implemented method according to claim 1, wherein the compressed values represent the spectral CT attenuation data corresponding to the one or more regions outside the identified one or more anatomical structures (150_{1..i}) with a relatively lower spatial resolution, or a relatively lower bit depth, than the spectral CT attenuation data corresponding to the identified one or more anatomical structures (150_{1..i}).

3. The computer-implemented method according to any one of claims 1 - 2, wherein the subset (140_{S}) of the spectral CT attenuation data corresponds to the one or more anatomical structures (150_{1..i}), and wherein:
the subset (140_{S}) of the spectral CT attenuation data represents at least one of the one or more anatomical structures (150_{1..i}) with a subset of the plurality of energy intervals (DE_{1..m}); or
the spectral CT attenuation data represents a plurality of different materials in the anatomical region, and wherein the subset (140_{S}) of the spectral CT attenuation data represents at least one of the one or more anatomical structures (150_{1..i}) with a subset of the materials represented by the spectral CT attenuation data.

4. The computer-implemented method according to claim 3, wherein the method further comprises:
applying a material decomposition algorithm to the spectral CT attenuation data to identify the one or more materials.

5. The computer-implemented method according to any previous claim, wherein the identifying one or more anatomical structures (150_{1..i}) in the spectral CT attenuation data, comprises performing a segmentation operation on the spectral CT attenuation data.

6. The computer-implemented method according to any previous claim, wherein the subset (140_{S}) of the spectral CT attenuation data is determined by the host processing system (130), or by the remote processing system (170); and
wherein the subset (140_{S}) is determined based on the identified one or more anatomical structures (150_{1..i}) using a look-up table, or a neural network; and
wherein the lookup table, or the neural network, provides for the one or more anatomical structures (150_{1..i}), a corresponding subset (140_{S}) of the spectral CT attenuation data required for calculating the value of the blood flow parameter.

7. The computer-implemented method according to claim 6, wherein the subset (140s) of the spectral CT attenuation data comprises a plurality of data elements, the data elements corresponding to different anatomical structures (150_{1..i}) and/or different materials in the anatomical region; and
wherein the method further comprises:
assigning, by the host processing system (130), or by the remote processing system (170), a transmission priority to the plurality of data elements in the subset; and
wherein the transferring the subset (140_{S}) of the spectral CT attenuation data comprises transferring the data elements in the order of the assigned transmission priority.

8. The computer-implemented method according to any previous claim, wherein the remote processing system (170) is further configured to:
predict a confidence value representing an expected accuracy of the calculated value (110) of the blood flow parameter; and
transfer the confidence value to the host processing system (130) over the communication channel (160).

9. The computer-implemented method according to any previous claim, wherein the remote processing system (170) is further configured to:
identify a type of the data in the subset (140_{S}) of the spectral CT attenuation data transferred to the remote processing system; and
select an algorithm for use by the remote processing system (170) to calculate the value (110) of the blood flow parameter, based on the identified type of the data; or
translate the subset (140_{S}) of the spectral attenuation CT data transferred to the remote processing system (170) into a format for use by the remote processing system to calculate the value (110) of the blood flow parameter, based on the identified type of the data.

10. The computer-implemented method according to any previous claim, wherein the anatomical region is a thoracic region;
and/or
wherein the one or more anatomical structures (150_{1..i}) include one or more of: an organ, a heart, an atrium of the heart, a myocardium of the heart, a lumen of at least one of the one or more vessels (120), a wall of one or more of the one or more vessels (120), a vessel plaque of one or more of the one or more vessels (120).

11. The computer-implemented method according to any previous claim, wherein the method further comprises:
receiving, by the host processing system (130), input identifying the one or more vessels (120) in the spectral CT attenuation data (140); and
wherein the value (110) of the blood flow parameter is calculated for the identified one or more vessels (120).

12. The computer-implemented method according to any previous claim, wherein the blood flow parameter is the fractional flow reserve, FFR, or the instantaneous wave-free ratio, iFR, or the index of myocardial resistance, IMR, or the coronary flow reserve, CFR.

13. A computer program product comprising instructions which when executed by one or more processors (310), cause the one or more processors to carry out the method according to any one of claims 1 - 12.

14. A system (300) for determining a value (110) of a blood flow parameter for one or more vessels (120), the system comprising one or more processors (310) configured to:
receive (S110) spectral computed tomography, CT, attenuation data (140) representing an injected contrast agent within an anatomical region including the one or more vessels (120), the spectral CT attenuation (140) data defining X-ray attenuation within the anatomical region in a plurality of different energy intervals (DE_{1..m});
identify (S120) one or more anatomical structures (150_{1..i}) in the spectral CT attenuation data (140);
determine (S130) a subset (140_{S}) of the spectral CT attenuation data (140) for use in calculating the value (110) of the blood flow parameter, based on the identified one or more anatomical structures (150_{1..i});
transfer (S140) the subset (140_{S}) of the spectral CT attenuation data over a communication channel (160) to a remote processing system (170), the remote processing system being configured to calculate the value (110) of the blood flow parameter from the subset of the spectral CT attenuation data; and
receive (S150) the value (110) of the blood flow parameter calculated by the remote processing system (170);
wherein:
the subset (140_{S}) of the spectral CT attenuation data includes only the spectral CT attenuation data corresponding to the identified one or more anatomical structures (150_{1..i}); or
the subset (140_{S}) comprises the spectral CT attenuation data corresponding to the identified one or more anatomical structures (150_{1..i}), and compressed or default values for the spectral CT attenuation data corresponding to one or more regions outside the identified one or more anatomical structures (150_{1..i}),

## Patentansprüche

1. Computerimplementiertes Verfahren zum Bestimmen eines Wertes (110) eines Blutflussparameters für ein oder mehrere Gefäße (120), wobei das Verfahren umfasst:
Empfangen (S110), durch ein Host-Verarbeitungssystem (130), von spektralen Computertomographie-, CT-, Schwächungsdaten (140), die ein injiziertes Kontrastmittel innerhalb einer anatomischen Region darstellen, die das eine oder die mehreren Gefäße (120) beinhaltet, wobei die spektralen CT-Schwächungsdaten (140) Röntgenschwächung innerhalb der anatomischen Region in einer Vielzahl von unterschiedlichen Energieintervallen (DE_{1..m}) definieren;
Identifizieren (S120), durch das Host-Verarbeitungssystem (130), einer oder mehrerer anatomischer Strukturen (150_{1..i}) in den spektralen CT-Schwächungsdaten (140);
Bestimmen (S130) einer Teilmenge (140s) der spektralen CT-Schwächungsdaten (140) zur Verwendung beim Berechnen des Wertes (110) des Blutflussparameters auf Basis der einen oder mehreren identifizierten anatomischen Strukturen (150_{1..i});
Übermitteln (S140) der Teilmenge (140_{S}) der spektralen CT-Schwächungsdaten von dem Host-Verarbeitungssystem (130) über einen Kommunikationskanal (160) an ein Fernverarbeitungssystem (170), wobei das Fernverarbeitungssystem so konfiguriert ist, dass es den Wert (110) des Blutflussparameters anhand der Teilmenge der spektralen CT-Schwächungsdaten berechnet; und
Empfangen (S150), durch das Host-Verarbeitungssystem (130), des Wertes (110) des Blutflussparameters, der von dem Fernverarbeitungssystem (170) berechnet wurde;
wobei:
die Teilmenge (140_{S}) der spektralen CT-Schwächungsdaten nur die spektralen CT-Schwächungsdaten beinhaltet, die der einen oder den mehreren identifizierten anatomischen Strukturen (150_{1..i}) entsprechen; oder
die Teilmenge (140_{S}) die spektralen CT-Schwächungsdaten, die der einen oder den mehreren identifizierten anatomischen Strukturen (150_{I..i}) entsprechen, und komprimierte oder Standardwerte für die spektralen CT-Schwächungsdaten umfasst, die einer oder mehreren Regionen außerhalb der einen oder mehreren identifizierten anatomischen Strukturen (150_{1..i}) entsprechen.

2. Computerimplementiertes Verfahren nach Anspruch 1, wobei die komprimierten Werte die spektralen CT-Schwächungsdaten, die der einen oder den mehreren Regionen außerhalb der einen oder mehreren identifizierten anatomischen Strukturen (150_{1..i}) entsprechen, mit einer relativ geringeren räumlichen Auflösung oder einer relativ geringeren Bittiefe darstellen als die spektralen CT-Schwächungsdaten, die der einen oder den mehreren identifizierten anatomischen Strukturen (150_{1..i}) entsprechen.

3. Computerimplementiertes Verfahren nach einem der Ansprüche 1-2, wobei die Teilmenge (140_{S}) der spektralen CT-Schwächungsdaten der einen oder den mehreren anatomischen Strukturen (150_{1..i}) entspricht, und wobei:
die Teilmenge (140_{S}) der spektralen CT-Schwächungsdaten mindestens eine der einen oder mehreren anatomischen Strukturen (150_{1..i}) mit einer Teilmenge der Vielzahl von Energieintervallen (DE_{1..m}) darstellt; oder
die spektralen CT-Schwächungsdaten eine Vielzahl von unterschiedlichen Materialien in der anatomischen Region darstellen, und wobei die Teilmenge (140_{S}) der spektralen CT-Schwächungsdaten mindestens eine der einen oder mehreren anatomischen Strukturen (150_{1..i}) mit einer Teilmenge der durch die spektralen CT-Schwächungsdaten dargestellten Materialien darstellt.

4. Computerimplementiertes Verfahren nach Anspruch 3, wobei das Verfahren weiter umfasst:
Anwenden eines Materialzerlegungsalgorithmus auf die spektralen CT-Schwächungsdaten, um das eine oder die mehreren Materialien zu identifizieren.

5. Computerimplementiertes Verfahren nach einem vorstehenden Anspruch, wobei das Identifizieren einer oder mehrerer anatomischer Strukturen (150_{1..i}) in den spektralen CT-Schwächungsdaten das Durchführen einer Segmentierungsoperation an den spektralen CT-Schwächungsdaten umfasst.

6. Computerimplementiertes Verfahren nach einem vorstehenden Anspruch, wobei die Teilmenge (140_{S}) der spektralen CT-Schwächungsdaten durch das Host-Verarbeitungssystem (130) oder durch das Fernverarbeitungssystem (170) bestimmt wird; und
wobei die Teilmenge (140s) auf Basis der einen oder mehreren identifizierten anatomischen Strukturen (150_{1..i}) unter Verwendung einer Nachschlagstabelle oder eines neuronalen Netzwerks bestimmt wird; und
wobei die Nachschlagstabelle oder das neuronale Netzwerk für die eine oder die mehreren anatomischen Strukturen (150_{1..i}) eine entsprechende Teilmenge (140_{S}) der spektralen CT-Schwächungsdaten bereitstellt, die zum Berechnen des Wertes des Blutflussparameters erforderlich ist.

7. Computerimplementiertes Verfahren nach Anspruch 6, wobei die Teilmenge (140_{S}) der spektralen CT-Schwächungsdaten eine Vielzahl von Datenelementen umfasst, wobei die Datenelemente unterschiedlichen anatomischen Strukturen (150_{1..i}) und/oder unterschiedlichen Materialien in der anatomischen Region entsprechen; und
wobei das Verfahren weiter umfasst:
Zuweisen, durch das Host-Verarbeitungssystem (130) oder durch das Fernverarbeitungssystem (170), einer Übertragungspriorität an die Vielzahl von Datenelementen in der Teilmenge; und
wobei das Übermitteln der Teilmenge (140_{S}) der spektralen CT-Schwächungsdaten das Übermitteln der Datenelemente in der Reihenfolge der zugewiesenen Übertragungspriorität umfasst.

8. Computerimplementiertes Verfahren nach einem vorstehenden Anspruch, wobei das Fernverarbeitungssystem (170) weiter so konfiguriert ist, dass es:
einen Konfidenzwert vorhersagt, der eine erwartete Genauigkeit des berechneten Wertes (110) des Blutflussparameters darstellt; und
den Konfidenzwert über den Kommunikationskanal (160) an das Host-Verarbeitungssystem (130) übermittelt.

9. Computerimplementiertes Verfahren nach einem vorstehenden Anspruch, wobei das Fernverarbeitungssystem (170) weiter so konfiguriert ist, dass es:
einen Typ der Daten in der Teilmenge (140_{S}) der spektralen CT-Schwächungsdaten, die an das Fernverarbeitungssystem übermittelt werden, identifiziert; und
einen Algorithmus zur Verwendung durch das Fernverarbeitungssystem (170), um den Wert (110) des Blutflussparameters zu berechnen, auf Basis des identifizierten Typs der Daten auswählt; oder
die Teilmenge (140_{S}) der spektralen CT-Schwächungsdaten, die an das Fernverarbeitungssystem (170) übermittelt werden, auf Basis des identifizierten Typs der Daten in ein Format zur Verwendung durch das Fernverarbeitungssystem, um den Wert (110) des Blutflussparameters zu berechnen, übersetzt.

10. Computerimplementiertes Verfahren nach einem vorstehenden Anspruch, wobei die anatomische Region eine Thoraxregion ist;
und/oder
wobei die eine oder mehreren anatomischen Strukturen (150_{I..i}) eines oder mehrere beinhalten von: einem Organ, einem Herz, einem Vorhof des Herzens, einem Myokard des Herzens, einem Lumen mindestens eines des einen oder der mehreren Gefäße (120), einer Wand eines oder mehrerer des einen oder der mehreren Gefäße (120), einer Gefäßplaque eines oder mehrerer des einen oder der mehreren Gefäße (120).

11. Computerimplementiertes Verfahren nach einem vorstehenden Anspruch, wobei das Verfahren weiter umfasst:
Empfangen, durch das Host-Verarbeitungssystem (130), einer Eingabe, die das eine oder die mehreren Gefäße (120) in den spektralen CT-Schwächungsdaten (140) identifiziert; und
wobei der Wert (110) des Blutflussparameters für das eine oder die mehreren identifizierten Gefäße (120) berechnet wird.

12. Computerimplementiertes Verfahren nach einem vorstehenden Anspruch, wobei der Blutflussparameter die fraktionelle Flussreserve, FFR, oder das Instantaneous Wave-Free Ratio, iFR, oder der Index des Myokardwiderstands, IMR, oder die koronare Flussreserve, CFR, ist.

13. Computerprogrammprodukt, das Anweisungen umfasst, die, wenn sie von einem oder mehreren Prozessoren (310) ausgeführt werden, den einen oder die mehreren Prozessoren veranlassen, das Verfahren nach einem der Ansprüche 1-12 auszuführen.

14. System (300) zum Bestimmen eines Wertes (110) eines Blutflussparameters für ein oder mehrere Gefäße (120), wobei das System einen oder mehrere Prozessoren (310) umfasst, die so konfiguriert sind, dass sie:
spektrale Computertomographie-, CT-, Schwächungsdaten (140) empfangen (S110), die ein injiziertes Kontrastmittel innerhalb einer anatomischen Region darstellen, welche das eine oder die mehreren Gefäße (120) beinhaltet, wobei die spektralen CT-Schwächungsdaten (140) Röntgenschwächung innerhalb der anatomischen Region in einer Vielzahl von unterschiedlichen Energieintervallen (DE_{1..m}) definieren;
eine oder mehrere anatomische Strukturen (150_{1..i}) in den spektralen CT-Schwächungsdaten (140) identifizieren (S120);
eine Teilmenge (140_{S}) der spektralen CT-Schwächungsdaten (140) zur Verwendung beim Berechnen des Wertes (110) des Blutflussparameters auf Basis der einen oder mehreren identifizierten anatomischen Strukturen (150_{1..i}) bestimmen (S130);
die Teilmenge (140_{S}) der spektralen CT-Schwächungsdatenüber einen Kommunikationskanal (160) an ein Fernverarbeitungssystem (170) übermitteln (S140), wobei das Fernverarbeitungssystem so konfiguriert ist, dass es den Wert (110) des Blutflussparameters anhand der Teilmenge der spektralen CT-Schwächungsdaten berechnet; und
den Wert (110) des Blutflussparameters, der von dem Fernverarbeitungssystem (170) berechnet wurde, empfangen (S150);
wobei:
die Teilmenge (140_{S}) der spektralen CT-Schwächungsdaten nur die spektralen CT-Schwächungsdaten beinhaltet, die der einen oder den mehreren identifizierten anatomischen Strukturen (150_{1..i}) entsprechen; oder
die Teilmenge (140_{S}) die spektralen CT-Schwächungsdaten, die der einen oder den mehreren identifizierten anatomischen Strukturen (150_{1..i}) entsprechen, und komprimierte oder Standardwerte für die spektralen CT-Schwächungsdaten umfasst, die einer oder mehreren Regionen außerhalb der einen oder mehreren identifizierten anatomischen Strukturen (150_{1..i}) entsprechen.

## Revendications

1. Procédé, mis en œuvre par ordinateur, de détermination d'une valeur (110) d'un paramètre de débit sanguin pour un ou plusieurs vaisseaux (120), le procédé comprenant :
la réception (S110), par un système de traitement hôte (130), de données d'atténuation de tomodensitométrie, CT, spectrale (140) représentant un agent de contraste injecté dans une région anatomique incluant les un ou plusieurs vaisseaux (120), les données d'atténuation CT spectrale (140) définissant une atténuation des rayons X à l'intérieur de la région anatomique dans une pluralité d'intervalles d'énergie (DE_{1..m}) différents ;
l'identification (S120), par le système de traitement hôte (130), d'une ou plusieurs structures anatomiques (150_{1..i}) dans les données d'atténuation CT spectrale (140) ;
la détermination (S130) d'un sous-ensemble (140s) des données d'atténuation CT spectrale (140) à utiliser pour calculer la valeur (110) du paramètre de débit sanguin, sur la base des une ou plusieurs structures anatomiques (150_{1..i}) identifiées ;
le transfert (S140) du sous-ensemble (140_{S}) des données d'atténuation CT spectrale du système de traitement hôte (130) via un canal de communication (160) vers un système de traitement distant (170), le système de traitement distant étant configuré pour calculer la valeur (110) du paramètre de débit sanguin à partir du sous-ensemble des données d'atténuation CT spectrale ; et
la réception (S150), par le système de traitement hôte (130), de la valeur (110) du paramètre de débit sanguin calculée par le système de traitement distant (170) ;
dans lequel :
le sous-ensemble (140s) des données d'atténuation CT spectrale inclut uniquement les données d'atténuation CT spectrale correspondant aux une ou plusieurs structures anatomiques (150_{1..i}) identifiées ; ou
le sous-ensemble (140_{S}) comprend les données d'atténuation CT spectrale correspondant aux une ou plusieurs structures anatomiques (150_{I..i}) identifiées, et des valeurs compressées ou par défaut pour les données d'atténuation CT spectrale correspondant à une ou plusieurs régions en dehors des une ou plusieurs structures anatomiques (150_{1..i}) identifiées.

2. Procédé mis en œuvre par ordinateur selon la revendication 1, dans lequel les valeurs compressées représentent les données d'atténuation CT spectrale correspondant aux une ou plusieurs régions en dehors des une ou plusieurs structures anatomiques (150_{1..i}) identifiées avec une résolution spatiale relativement inférieure, ou une profondeur de bits relativement inférieure, par rapport aux données d'atténuation CT spectrale correspondant aux une ou plusieurs structures anatomiques (150_{1..i}) identifiées.

3. Procédé mis en œuvre par ordinateur selon l'une quelconque des revendications 1-2, dans lequel le sous-ensemble (140s) des données d'atténuation CT spectrale correspond aux une ou plusieurs structures anatomiques (150_{1..i}), et dans lequel :
le sous-ensemble (140s) des données d'atténuation CT spectrale représente au moins une des une ou plusieurs structures anatomiques (150_{1..i}) avec un sous-ensemble de la pluralité d'intervalles d'énergie (DE_{1..m).}) ; ou
les données d'atténuation CT spectrale représentent une pluralité de matières différentes dans la région anatomique, et dans lequel le sous-ensemble (140_{S}) des données d'atténuation CT spectrale représente au moins une des une ou plusieurs structures anatomiques (150_{1..i}) avec un sous-ensemble des matières représentées par les données d'atténuation CT spectrale.

4. Procédé mis en œuvre par ordinateur selon la revendication 3, dans lequel le procédé comprend en outre :
l'application d'un algorithme de décomposition de matières aux données d'atténuation CT spectrale pour identifier les une ou plusieurs matières.

5. Procédé mis en œuvre par ordinateur selon une quelconque revendication précédente, dans lequel l'identification d'une ou plusieurs structures anatomiques (150_{1..i}) dans les données d'atténuation CT spectrale comprend la réalisation d'une opération de segmentation sur les données d'atténuation CT spectrale.

6. Procédé mis en œuvre par ordinateur selon une quelconque revendication précédente, dans lequel le sous-ensemble (140_{S}) des données d'atténuation CT spectrale est déterminé par le système de traitement hôte (130), ou par le système de traitement distant (170) ; et
dans lequel le sous-ensemble (140_{S}) est déterminé sur la base des une ou plusieurs structures anatomiques (150_{1..i}) identifiées à l'aide d'une table de consultation ou d'un réseau neuronal ; et
dans lequel la table de consultation, ou le réseau neuronal, fournit, pour les une ou plusieurs structures anatomiques (150_{1..i}), un sous-ensemble (140_{S}) correspondant des données d'atténuation CT spectrale nécessaires au calcul de la valeur du paramètre de débit sanguin.

7. Procédé mis en œuvre par ordinateur selon la revendication 6, dans lequel le sous-ensemble (140s) des données d'atténuation CT spectrale comprend une pluralité d'éléments de données, les éléments de données correspondant à différentes structures anatomiques (150_{1..i}) et/ou à différentes matières dans la région anatomique ; et
dans lequel le procédé comprend en outre :
l'attribution, par le système de traitement hôte (130) ou par le système de traitement distant (170), d'une priorité de transmission à la pluralité d'éléments de données dans le sous-ensemble ; et
dans lequel le transfert du sous-ensemble (140_{S}) des données d'atténuation CT spectrale comprend le transfert des éléments de données dans l'ordre de la priorité de transmission attribuée.

8. Procédé mis en œuvre par ordinateur selon une quelconque revendication précédente, dans lequel le système de traitement distant (170) est en outre configuré pour :
prédire une valeur de confiance représentant une précision attendue de la valeur calculée (110) du paramètre de débit sanguin ; et
transférer la valeur de confiance au système de traitement hôte (130) via le canal de communication (160).

9. Procédé mis en œuvre par ordinateur selon une quelconque revendication précédente, dans lequel le système de traitement distant (170) est en outre configuré pour :
identifier un type de données dans le sous-ensemble (140s) des données d'atténuation CT spectrale transférées au système de traitement distant ; et
sélectionner un algorithme à utiliser par le système de traitement distant (170) pour calculer la valeur (110) du paramètre de débit sanguin, sur la base du type de données identifié ; ou
traduire le sous-ensemble (140_{S}) des données CT d'atténuation spectrale transférées au système de traitement distant (170) dans un format utilisable par le système de traitement distant pour calculer la valeur (110) du paramètre de débit sanguin, sur la base du type de données identifié.

10. Procédé mis en œuvre par ordinateur selon une quelconque revendication précédente, dans lequel la région anatomique est une région thoracique ;
et/ou
dans lequel les une ou plusieurs structures anatomiques (150_{1..i}) incluent un ou plusieurs parmi : un organe, un cœur, une oreillette du cœur, un myocarde du cœur, une lumière d'au moins un des un ou plusieurs vaisseaux (120), une paroi d'un ou plusieurs des un ou plusieurs vaisseaux (120), une plaque de vaisseau d'un ou plusieurs des un ou plusieurs vaisseaux (120).

11. Procédé mis en œuvre par ordinateur selon une quelconque revendication précédente, dans lequel le procédé comprend en outre :
la réception, par le système de traitement hôte (130), d'une entrée identifiant les un ou plusieurs vaisseaux (120) dans les données d'atténuation CT spectrale (140) ; et
dans lequel la valeur (110) du paramètre de débit sanguin est calculée pour les un ou plusieurs vaisseaux (120) identifiés.

12. Procédé mis en œuvre par ordinateur selon une quelconque revendication précédente, dans lequel le paramètre de débit sanguin est la réserve de débit fractionnaire, FFR, ou le rapport instantané sans onde, iFR, ou l'indice de résistance myocardique, IMR, ou la réserve de débit coronaire, CFR.

13. Produit de programme informatique comprenant des instructions qui, lorsqu'elles sont exécutées par un ou plusieurs processeurs (310), amènent les un ou plusieurs processeurs à réaliser la procédé selon l'une quelconque des revendications 1-12.

14. Système (300) de détermination d'une valeur (110) d'un paramètre de débit sanguin pour un ou plusieurs vaisseaux (120), le système comprenant un ou plusieurs processeurs (310) configurés pour :
recevoir (S110) des données d'atténuation de tomodensitométrie, CT, spectrale (140) représentant un agent de contraste injecté dans une région anatomique incluant les un ou plusieurs vaisseaux (120), les données d'atténuation CT spectrale (140) définissant l'atténuation des rayons X dans la région anatomique dans une pluralité d'intervalles d'énergie (DE_{1..m).}) différents ;
identifier (S120) une ou plusieurs structures anatomiques (150_{1..i}) dans les données d'atténuation CT spectrale (140) ;
déterminer (5130) un sous-ensemble (140s) des données d'atténuation CT spectrale (140) à utiliser pour calculer la valeur (110) du paramètre de débit sanguin, sur la base des une ou plusieurs structures anatomiques (150_{1..i}) identifiées ;
transférer (S140) le sous-ensemble (140_{S}) des données d'atténuation CT spectrale via un canal de communication (160) vers un système de traitement distant (170), le système de traitement distant étant configuré pour calculer la valeur (110) du paramètre de débit sanguin à partir du sous-ensemble des données d'atténuation CT spectrale ; et
recevoir (S150) la valeur (110) du paramètre de débit sanguin calculé par le système de traitement distant (170) ;
dans lequel :
le sous-ensemble (140s) des données d'atténuation CT spectrale inclut uniquement les données d'atténuation CT spectrale correspondant aux une ou plusieurs structures anatomiques (150_{1..i}) identifiées ; ou
le sous-ensemble (140_{S}) comprend les données d'atténuation CT spectrale correspondant aux une ou plusieurs structures anatomiques (150_{1..i}) identifiées, et des valeurs compressées ou par défaut pour les données d'atténuation CT spectrale correspondant à une ou plusieurs régions en dehors des une ou plusieurs structures anatomiques (150_{1..i}) identifiées.
